(19) Europäisches Patentamt
European Patent Office
Office européen des brevets

(11) EP 1 235 787 B1

(12) EUROPEAN PATENT SPECIFICATION

(45) Date of publication and mention of the grant of the patent:
**29.10.2003 Bulletin 2003/44**

(21) Application number: **00986271.5**

(22) Date of filing: **06.12.2000**

(51) Int Cl.[7]: **C07C 233/43**, C07C 215/68, A61K 31/135, A61K 31/165, A61P 11/00, A61P 25/00

(86) International application number:
**PCT/US00/33057**

(87) International publication number:
**WO 01/042193 (14.06.2001 Gazette 2001/24)**

# (54) $g(b)2-ADRENERGIC RECEPTOR AGONISTS

BETA 2 -ADRENERGISCHE REZEPTOR-AGONISTEN

AGONISTES DES RECEPTEURS ADRENERGIQUES DU BETA 2

(84) Designated Contracting States:
**AT BE CH CY DE DK ES FI FR GB GR IE IT LI LU MC NL PT SE TR**
Designated Extension States:
**AL LT LV MK RO SI**

(30) Priority: **08.12.1999 US 457618**
**14.08.2000 US 637899**

(43) Date of publication of application:
**04.09.2002 Bulletin 2002/36**

(73) Proprietor: **Theravance, Inc.**
**South San Francisco, CA 94080 (US)**

(72) Inventors:
- **MORAN, Edmund, J.**
  **San Francisco, CA 94127 (US)**
- **CHOI, Seok-Ki**
  **Palo Alto, CA 94301 (US)**

(74) Representative: **Scott, Susan Margaret et al**
**Abel & Imray,**
**20 Red Lion Street**
**London WC1R 4PQ (GB)**

(56) References cited:
**WO-A-98/21175**
**WO-A-99/64035**
**GB-A- 1 040 724**
**GB-A- 1 394 542**

- **PATENT ABSTRACTS OF JAPAN vol. 1998, no. 11, 30 September 1998 (1998-09-30) & JP 10 152460 A (KISSEI PHARMACEUT CO LTD), 9 June 1998 (1998-06-09) cited in the application**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### Field of the Invention

This invention relates to novel multibinding compounds (agents) that are β2 adrenergic receptor agonists, partial agonists and pharmaceutical compositions comprising such compounds. Accordingly, the multibinding compounds and pharmaceutical compositions of this invention are useful in the treatment and prevention of respiratory diseases such as asthma, chronic obstructive pulmonary disease and chronic bronchitis. They are also useful in the treatment of nervous system injury and premature labor.

### References

The following publications are cited in this application as superscript numbers:

[1] Hardman, J. G., et al. "The Pharmacological Basis of Therapeutics", McGraw-Hill, New York, (1996)

[2] Strosberg, A. D. "Structure, Function, and Regulation of Adrenergic Receptors" *Protein Sci.* 2, 1198-1209 (1993).

[3] Beck-Sickinger, A. G. "Structure Characterization and Binding Sites of G-Protein-coupled Receptors" *DDT*, 1, 502-513, (1996).

[4] Hein, L. & Kobilka, B. K. "Adrenergic Receptor Signal Transduction and Regulation" *Neuropharmacol*, 34, 357-366, (1995).

[5] Strosberg, A. D. & Pietri-Rouxel, F. "Function, and Regulation of β3-Adrenoceptor" *TiPS*, 17, 373-381, (1996).

[6] Barnes, P. J. "Current Therapies for Asthma" *CHEST,* 111:17S-26S, (1997).

[7] Jack, D. A. "A way of Looking at Agonism and Antagonism: Lessons from Salbutamol, Salmeterol and other β-Adrenoceptor Agonists" *Br. J. Clin. Pharmac*. 31, 501-514, (1991).

[8] Kissei Pharmaceutical Co. Ltd. "2-Amino-1-(4-hydroxy-2-methylphenyl)propanol derivatives" JP-10152460 (Publication date June 9, 1998).

All of the above publications are herein incorporated by reference in their entirety to the same extent as if each individual publication was specifically and individually indicated to be incorporated by reference in its entirety.

### State of the Art

A receptor is a biological structure with one or more binding domains that reversibly complexes with one or more ligands, where that complexation has biological consequences. Receptors can exist entirely outside the cell (extracellular receptors), within the cell membrane (but presenting sections of the receptor to the extracellular milieu and cytosol), or entirely within the cell (intracellular receptors). They may also function independently of a cell (e.g., clot formation). Receptors within the cell membrane allow a cell to communicate with the space outside of its boundaries (i.e., signaling) as well as to function in the transport of molecules and ions into and out of the cell.

A ligand is a binding partner for a specific receptor or family of receptors. A ligand may be the endogenous ligand for the receptor or alternatively may be a synthetic ligand for the receptor such as a drug, a drug candidate or a pharmacological tool.

The super family of seven transmembrane proteins (7-TMs), also called G-protein coupled receptors (GPCRs), represents one of the most significant classes of membrane bound receptors that communicate changes that occur outside of the cell's boundaries to its interior, triggering a cellular response when appropriate. The G-proteins, when activated, affect a wide range of downstream effector systems both positively and negatively (e.g., ion channels, protein kinase cascades, transcription, transmigration of adhesion proteins, and the like).

Adrenergic receptors (AR) are members of the G-protein coupled receptors that are composed of a family of three receptor sub-types: $\beta 1_{(A, B, D)}$ $\beta 2_{(A, B, C)}$, and $\beta_{(1, 2, 3)}$.[1-5] These receptors are expressed in tissues of various systems and organs of mammals and the proportions of the α and the β receptors are tissue dependant. For example,

tissues of bronchial smooth muscle express largely β2-AR while those of cutaneous blood vessels contain exclusively β-AR subtypes.

It has been established that the β2-AR sub-type is involved in respiratory diseases such as such as asthma[6], chronic bronchitis, nervous system injury, and premature labor[8]. Currently, a number of drugs e.g., albuterol, formoterol, isoprenolol, or salmeterol having β2-AR agonist activities are being used to treat asthma. However, these drugs have limited utility as they are either non-selective thereby causing adverse side effects such as muscle tremor, tachycardia, palpitations, and restlesness[6], or have short duration of action and/or slow onset time of action.[7] Accordingly, there is a need for β2-selective AR agonists that are fast acting and have increased potency and /or longer duration of action.

The multibinding compounds of the present invention fulfill this need.

Various agonists or partial agonists of β2 adrenergic receptors are known. WO 98/21175 discloses a method of preparation of optically pure isomers of formoterol, an agonist having a long lasting bronchodilating effect when inhaled. GB-A-1394542 relates to optically active isomers of certain phenyl hydroxyamines showing a selective effect on β2 receptors. GB-A-1040724 discloses novel amino alcohol compounds valuable in the treatment of heart or circulatory diseases, and a method for their preparation.

## SUMMARY OF THE INVENTION

This invention is directed to novel multibinding compounds (agents) that are agonists or partial agonists of β2 adrenergic receptor and are therefore useful in the treatment and prevention of respiratory diseases such as asthma, chronic obstructive pulmonary disease, and chronic bronchitis. They are also useful in the treatment of nervous system injury and premature labor.

Accordingly, the present invention provides a compound of the formula

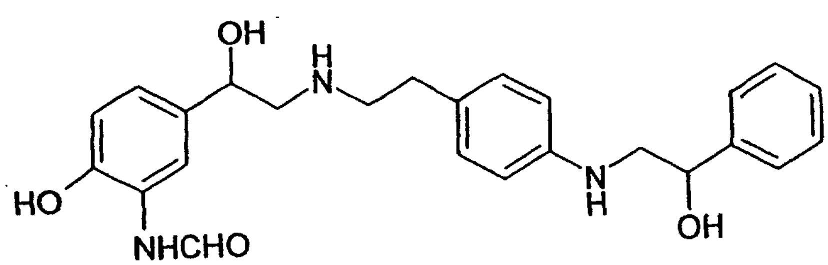

or a pharmaceutically acceptable salt thereof. The invention further provides a pharmaceutical composition comprising a pharmaceutically acceptable carrier and a therapeutically effective amount of a compound according to the invention; a compound or a composition according to the invention for use as a medicament; and the use of a compound or a composition according to the invention in the manufacture of a medicament for treating a disease mediated by a β2 adrenergic receptor in a mammal.

## BRIEF DESCRIPTION OF THE DRAWING

FIG. 1 illustrates the synthesis of a compound according to the invention.

## DETAILED DESCRIPTION OF THE INVENTION

Definitions

This invention is directed to multibinding compounds which are β2 adrenergic receptor agonists, pharmaceutical compositions containing such compounds and methods for treating diseases mediated by β2 adrenergic receptor in mammals. When discussing such compounds, compositions or methods, the following terms have the following meanings unless otherwise indicated. Any undefined terms have their art recognized meanings.

The term "pharmaceutically-acceptable salt" refers to salts which retain the biological effectiveness and properties of the multibinding compounds of this invention and which are not biologically or otherwise undesirable.

Pharmaceutically-acceptable base addition salts can be prepared from inorganic and organic bases. Salts derived from inorganic bases, include by way of example only, sodium, potassium, lithium, ammonium, calcium and

magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group. Examples of suitable amines include, by way of example only, isopropylamine, trimethyl amine, diethyl amine, tri(*iso*-propyl) amine, tri(*n*-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, *p*-toluene-sulfonic acid, salicylic acid, naphthoic acid, 2-hydroxynaphthoic acid, and the like.

The term "pharmaceutically-acceptable cation" refers to the cation of a pharmaceutically-acceptable salt.

The term "protecting group" or "blocking group" refers to any group which when bound to one or more, amino groups of the compounds (including intermediates thereof) prevents reactions from occurring at these groups and which protecting group can be removed by conventional chemical or enzymatic steps to reestablish the amino group (*See.,* T.W. Greene and P.G.H. Wuts, *"Protective Groups in Organic Synthesis",* 2nd Ed.). The particular removable blocking group employed is not critical.

Preferred removable amino blocking groups include conventional substituents such as t-butyoxycarbonyl (t-BOC), benzyloxycarbonyl (CBZ), fluorenylmethoxy-carbonyl (FMOC), allyloxycarbonyl (ALOC), and the like which can be removed by conventional conditions compatible with the nature of the product.

The term "treatment" refers to any treatment of a pathologic condition in a mammal, particularly a human, and includes:

(i) preventing the pathologic condition from occurring in a subject which may be predisposed to the condition but has not yet been diagnosed with the condition and, accordingly, the treatment constitutes prophylactic treatment for the disease condition;
(ii) inhibiting the pathologic condition, i.e., arresting its development;
(iii) relieving the pathologic condition, i.e., causing regression of the pathologic condition; or
(iv) relieving the conditions mediated by the pathologic condition.

The term "therapeutically effective amount" refers to that amount of multibinding compound which is sufficient to effect treatment, as defined above, when administered to a mammal in need of such treatment. The therapeutically effective amount will vary depending upon the subject and disease condition being treated, the weight and age of the subject, the severity of the disease condition, the manner of administration and the like, which can readily be determined by one of ordinary skill in the art.

The compounds of the invention exist in stereoisomeric form;

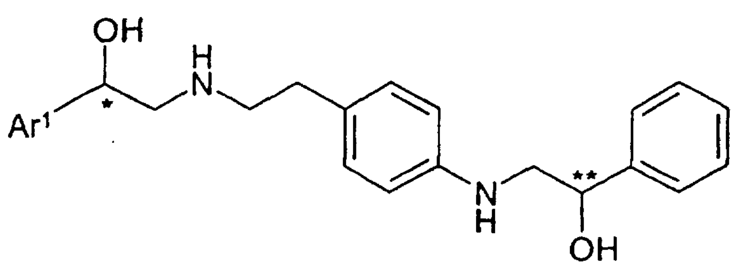

thus typically the stereochemistry at *C is (*RS*) and the stereochemistry at **C is (*RS*) or the stereochemistry at *C is (*R*) and the stereochemistry at **C is (*R*) or the stereochemistry at *C is (*S*) and the stereochemistry at **C is (*S*) or the stereochemistry at *C is (*R*) and the stereochemistry at **C is (*S*) or the stereochemistry at *C is (*S*) and the stereochemistry at **C is (*R*). $Ar^1$ in the above formula is 3-formylamino-4-hydroxyphenyl.

## GENERAL SYNTHETIC SCHEME

Compounds of this invention can be made by the method depicted in the reaction scheme shown below.

The starting materials and reagents used in preparing these compounds are either available from commercial suppliers such as Aldrich Chemical Co., (Milwaukee, Wisconsin, USA), Bachem (Torrance, California, USA), Emka-Chemie, or Sigma (St. Louis, Missouri, USA) or are prepared by methods known to those skilled in the art following procedures set forth in references such as Fieser and Fieser's Reagents for Organic Synthesis, Volumes 1-15 (John Wiley and Sons, 1991); Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989).

The starting materials and the intermediates of the reaction may be isolated and purified if desired using conventional techniques, including but not limited to filtration, distillation, crystallization, chromatography, and the like. Such materials may be characterized using conventional means, including physical constants and spectral data.

Furthermore, it will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and G. M. Wuts, *Protecting Groups in Organic Synthesis,* Second Edition, Wiley, New York, 1991, and references cited therein.

This scheme is merely illustrative of some methods by which the compounds of this invention can be synthesized, and various modifications to this scheme can be made and will be suggested to one skilled in the art having referred to this disclosure.

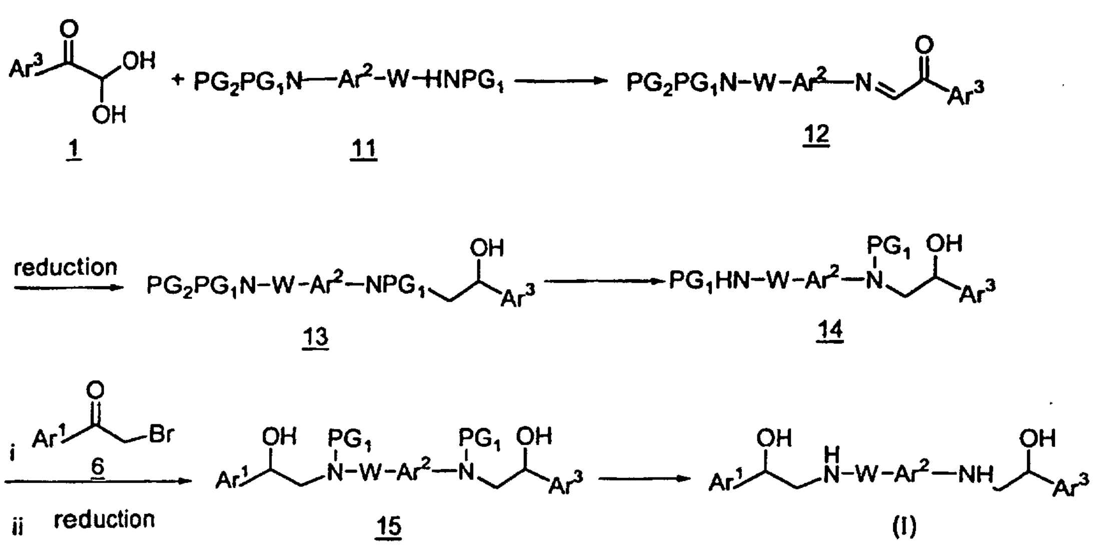


1 11 12 13 14 15 (I)

In this scheme, $Ar^1$ represents 4-hydroxy-3-formylaminophenyl; $Ar^2$ represents 1,4-phenylene; $Ar^3$ represents phenyl; and W represents $-CH_2-CH_2-$.

Condensation of a diamine of formula 11 (where $PG_1$ and $PG_2$ are suitable amino protecting groups which can be selectively removed) with a glyoxal of formula 2 followed by reduction of the resulting imine of formula 12 with a suitable reducing agent such as diborane in a suitable reaction solvents such as tetrahydrofuran provides a compound of formula 13. Compounds of formula 11 can be prepared by methods described in figure 14.

Reaction of compound 15 with an alpha bromoacetophenone compound of formula 6 followed by reduction

of the keto group provides a compound of formula 16. The reaction is carried out under conditions well known in the art. Deprotection of the amino protecting group then provides a compound of Formula (I). The deprotection reaction conditions depend on the nature of the protecting group. For example, if the protecting group is benzyl, it is removed under catalytic hydrogenation reaction conditions.

## Utility, Testing, and Administration

### Utility

The multibinding compounds of this invention are β2 adrenergic receptor agonists or partial agonists. Accordingly, the multibinding compounds and pharmaceutical compositions of this invention are useful in the treatment and prevention of diseases mediated by β2 adrenergic receptor such as asthma, chronic obstructive pulmonary disease, bronchitis, and the like. They are also useful in the treatment of nervous system injury and premature labor. It is also contemplated that the compounds of this invention are useful for treating metabolic disorders such as obesity, diabetes, and the like.

### Testing

The β2 adrenergic receptor agonistic activity of the compounds of Formula (I) to may be demonstrated by a variety of *in vitro* assays known to those of ordinary skill in the art, such as the assay described in the biological Examples 1 and 2. It may also be assayed by the *ex vivo* assays described in Ball, D. I. et al., "Salmeterol a Novel, Long-acting beta 2-Adrenergic Agonist: Characterization of Pharmacological Activity *in Vitro* and *in Vivo*" *Br. J. Pharmacol.*, 104, 665-671 (1991); Linden, A. et al., "Salmeterol, Formoterol, and Salbutamol in the Isolated Guinea-Pig Trachea: Differences in Maximum Relaxant Effect and Potency but not in Functional Atagonism. *Thorax*, 48, 547-553, (1993); and Bials, A. T. et al., Investigations into Factors Determining the Duration of Action of the Beta 2-Adrenoceptor Agonist, Salmeterol. *Br. J. Pharmacol.*, 108, 505-515 (1993); or *in vivo* assays such as those described in Ball, D. I. et al., "Salmeterol a Novel, Long-acting beta 2-Adrenergic Agonist: Characterization of Pharmacological Activity in *Vitro* and *in Vivo*" *Br. J. Pharmacol.*, 104, 665-671 (1991); Kikkawa, H. et al., "TA-2005, a Novel, Long-acting, and Selective Beta 2-Adrenoceptor Agonist: Characterization of its *in vivo* Bronchodilating Action in Guinea Pigs and Cats in Comparison with other Beta 2-Agonists". *Biol. Pharm. Bull.*, 17, 1047-1052, (1994); and Anderson, G. P., "Formoterol: Pharmacology, Molecular basis of Agonism and Mechanism of Long Duration of a Highly Potent and Selective Beta 2-Adrenoceptor Agonist Bronchodilator, *Life Sciences,* 52, 2145-2160, (1993).

### Pharmaceutical Formulations

When employed as pharmaceuticals, the compounds of this invention are usually administered in the form of pharmaceutical compositions. These compounds can be administered by a variety of routes including oral, rectal, transdermal, subcutaneous, intravenous, intramuscular, and inhalation (e.g., intranasal or oral inhalation). These compounds are effective as injectable, inhaled and oral compositions. Such compositions are prepared in a manner well known in the pharmaceutical art and comprise at least one active compound. A preferred manner for administering compounds of this invention is inhalation. This is an effective means for delivering a therapeutic agent directly to the respiratory tract for the treatment of diseases such as asthma and other similar or related respiratory tract disorders (*see* U. S. Patent No. 5,607,915).

This invention also includes a pharmaceutical composition which contains, as the active ingredient, a therapeutically effective amount of a compound according to the invention associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Preferably, the compound of Formula (I) above is employed at no more than about 20 weight percent of the pharmaceutical composition, more preferably no more than about 15 weight percent, with the balance being pharmaceutically inert carrier(s).

The active compound is effective over a wide dosage range and is generally administered in a pharmaceutically effective amount. For example, when the drug is administered via inhalation, each dosage contains from about 1 μg to about 1000 μg, preferably about 2 μg to about 500 μg, more preferably about 5 μg to about 100 μg, even more preferably about 5 μg to about 60 μg, of the active ingredient . It, will be understood, however, that the amount of the compound actually administered will be determined by a physician, in the light of the relevant circumstances, including the condition to be treated, the chosen route of administration, the actual compound administered and its relative activity, the age, weight, and response of the individual patient, the severity of the patient's symptoms, and the like. Furthermore, the compound of this invention may be administered prophylactically, for example, a pharmaceutical composition containing a compound of this invention may be administered before the bronchospasm begins in an asthma attack, to prevent its occurrence or to reduce the extent to which it occurs.

For preparing solid compositions such as tablets, the principal active ingredient is mixed with a pharmaceutical excipient to form a solid preformulation composition containing a homogeneous mixture of a compound of the present invention. When referring to these pre-formulation compositions as homogeneous, it is meant that the active ingredient is dispersed evenly throughout the composition so that the composition may be readily subdivided into equally effective unit dosage forms such as tablets, pills and capsules. This solid pre-formulation is then subdivided into unit dosage forms of the type described above containing the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders (*see* U. S. Patent No. 5,983,956). The liquid or solid compositions may contain suitable pharmaceutically acceptable excipients as described *supra*. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner (*see* U.S. Patents Nos. 5,919,827 and 5,972,919).

Furthermore, the pharmaceutical compositions containing one or more compound(s) of this invention can be administered in combination any other suitable drug, for example, with a suitable steroidal anti-inflammatory drug, e. g., budesonide, flucatisone, beclamethasone, for the treatment of respiratory disorders. When the combination therapy is employed, the pharmaceutical composition containing the compound(s) of this invention and the steroidal anti-inflammatory drug may be administered simultaneously, sequentially or separately. Each component used in the combination therapy is employed in an amount sufficient for its intended purpose. For example, the steriodal anti-inflammatory drugs are employed in sufficient amounts to effect reduction in inflammation *in vivo*. The β2 adrenergic receptor agonist/partial agonist compounds of this invention are employed in an amount sufficient to cause relaxation of smooth muscle tissue, for example, in the bronchial system.

Preferably, the dose range for compounds of this invention is from about 1 μg to about 1000 μg per dose, more preferably about 2 μg to about 500 μg, even more preferably about 5 μg to about 100 μg, and still more preferably

about 5 μg to about 60 μg. The preferred dosage range for a steroidal anti-inflammatory drug is from about 50 to 4800 μg and more preferably about 100 μg to about 1600 μg. Again, the particular dose used will depend on the patient (age, weight, etc.), and the severity of the disease (mild, moderate, severe). Lastly, a pharmaceutical composition containing the two active ingredients can also be prepared for administering the drugs simultaneously.

## EXAMPLES

The following preparations and examples are given to enable those skilled in the art to more clearly understand and to practice the present invention. They should not be considered as limiting the scope of the invention, but merely as being illustrative and representative thereof.

In the examples below, the following abbreviations have the following meanings. Unless otherwise stated, all temperatures are in degrees Celsius. If an abbreviation is not defined, it has its generally accepted meaning.

| | |
|---|---|
| Å = | Angstroms |
| cm = | centimeter |
| DCC = | dicyclohexyl carbodiimide |
| DMF = | *N,N*-dimethylformamide |
| DMSO = | dimethylsulfoxide |
| g = | gram |
| HPLC = | high performance liquid chromatography |
| MEM = | minimal essential medium |
| mg = | milligram |
| MIC = | minimum inhibitory concentration |
| min = | minute |
| mL = | milliliter |
| mm = | millimeter |
| mmol = | millimol |
| N = | normal |
| THF = | tetrahydrofuran |
| μL = | microliters |
| μm = | microns |
| rt = | room temperature |
| $R_f$ = | retention faction |
| NMR = | nuclear magnetic resonance |
| ESMS = | electrospray mass spectrum |
| ppm = | parts per million |

### Synthesis Example

Synthesis of 1-{2-[*N*-2-(4-hydroxy-3-formylaminophenyl)-2-(*RS*)-hydroxyethyl]aminoethyl}-4-[*N*-(2-phenyl-2-(*RS*)-hydroxyethyl)amino]phenyl (following figure 1)

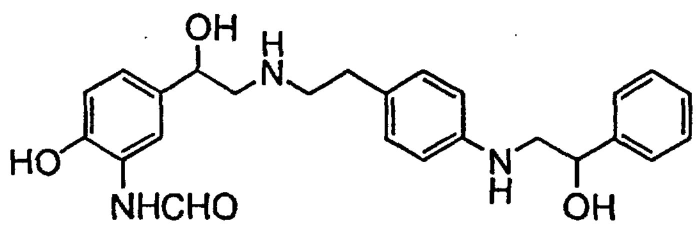

Step 1

A solution of 2-(4-aminophenyl)ethylamine **25** (4.70 mL, 36.7 mmol), benzaldehyde (7.46 mL, 73.4 mmol) and 4A molecular sieves (18 g) in toluene (180 mL) was refluxed for 3 h. The reaction mixture was cooled and filtered. The filtrate was concentrated under reduced pressure to afford 1-(4-benzyliminophenyl)-2-benzylimioethane (95% yield).

Step 2

To a cooled a solution of 1-(4-benzyliminophenyl)-2-benzyliminoethane (2.00 g, 6.40 mmol) in ethanol (150 mL) in ice bath was slowly added sodium borohydride (361 mg, 9.50 mmol) under a nitrogen atmosphere. The reaction mixture was allowed to stir at 0 °C for 1.5 h, and then warmed slowly to room temperature. The reaction mixture was quenched by slowly adding 50% methanol/TFA (5 mL) and then the mixture was concentrated under reduced pressure. The resultant residue was dissolved in EtOAc, and washed with 0.1 M NaOH. After drying over $Na_2SO_4$, the organic phase was concentrated *in vacuo*, and the residue was purified by flash silica gel chromatography using ethyl acetate/ hexanes as eluant to give compound 59(80% yield).

Step 3

1-Benzyloxy-4-bromoacetyl-2-nitrobenzene **66** (3.76 g, 10.8 mmol) [prepared as described in Chem. Bull., 25, 1368-1377, (1977)], was added to a solution of compound **59** (3.4 g, 10.8 mmol) in dimethylformamide (150 mL) at room temperature. After 28 h, the reaction mixture was diluted with ether and washed with a dilute solution of aqueous sodium chloride. The organic layer was separated and dried over sodium sulfate, filtered and concentrated to give a crude oil. Purification with column chromatography using hexane:ethyl acetate as the eluent provided compound **67** (95% yield).

Step 4

To a solution of **67** (4.0 g, 6.80 mmol) in ethanol (500 mL) was slowly added sodium borohydride (1 g, 26.50 mmol) in portions over 30 min., under a nitrogen atmosphere. After 6 h, the reaction mixture was quenched by slowly adding saturated solution of aqueous ammonium chloride . The solution was diluted with 1 N sodium hydroxide and ethyl acetate and hexanes. The organic layer was separated, dried over sodium sulfate, filtered and concentrated to give compound **68** as an oil which was used in the next step without further purification.

Step 5

To a mixture of **68** (3.6 g, 6.1 mmol) and potassium carbonate (3.0 g, 9.2 mmol) in dimethylformamide (100 mL) was added alpha-bromoacetophenone (1.28 g, 6.4 mmol) portionwise. The reaction mixture was heated overnight at 65 °C. An additional portion of alpha-bromoacetophenone (0.25 g, 1.25 mmol) was added and heating was continued. After 18 h, the reaction mixture was cooled to room temperature and ethanol (50 mL) was added. Sodium borohydride (1.0 g, 26.5 mmol) was added and stirring was continued for 2.5 h. The reaction mixture was concentrated and then methanol (25 mL) was added and the excess hydride was quenched with the addition of a saturated solution of ammonium chloride. The reaction mixture was diluted with ethyl acetate and ether. The organic layer was separated, dried over sodium sulfate, filtered, and concentrated. The crude oil was purified with column chromatography eluting with ethyl acetate/hexanes mixture to give compound **69**.

Step 6

To a solution of **69** (0.73 g, 1.0 mmol) in a mixture of methanol (20 mL), 6 N hydrochloride acid (1 mL) and water (2 mL) was added iron powder (0.56 g, 10.0 mmol). The reaction mixture was heated at 90 °C for 1.5 h. The reaction mixture was cooled to room temperature and allowed to stand overnight. Methanol was added and the brown precipitates and unreacted iron was filtered off. The filtrate was concentrated under reduced pressure, to give **70** as a brown solid which was used in the next step without further purification.

Step 7

Compound **70** was dissolved in a premixed solution of acetic anhydride (5 mL) and formic acid (3 mL) at room temperature. After 3 h, the reaction mixture was diluted with ethyl acetate and evaporated to dryness. A methanolic

solution of 0.5 M sodium hydroxide was added and the reaction mixture was stirred for 6 h at room temperature. The reaction mixture was treated with methanolic solution of 1 N hydrochloric acid and then concentrated to dryness. The residue was redissolved in methanol and filtered. The filtrate was concentrated to give compound **71** as a brown residue which was used in the next step without further purification.

Step 8

Palladium on carbon (10%, 0.5 g) was added to a suspension of **71** in methanol (120 mL) and dimethylformamide (80 mL). The reaction mixture was purged with nitrogen gas and stirred overnight under hydrogen atmosphere (1 atm) at room temperature. The mixture was filtered, and the filtrate was concentrated to yield crude product which was purified by HPLC (acetonitril/water/1% TFA gradient) to give crude product which was purified with column chromatography using methanol/methylene chloride/1%isopropylamine as the eluent to give desired compound 1-{2-[*N*-2-(4-hydroxy-3-formylaminophenyl)-2-(*RS*)-hydroxyethyl]-aminoethyl}-4-[*N*-(2-phenyl-2-(*RS*)-hydroxyethyl)amino] phenyl **72**.

Formulation Examples

Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

Example 2

A tablet Formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Example 3

A dry powder inhaler formulation is prepared containing the following components:

| Ingredient | Weight % |
|---|---|
| Active Ingredient | 5 |
| Lactose | 95 |

The active ingredient is mixed with the lactose and the mixture is added to a dry powder inhaling appliance.

Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in sterile water) | 4.0 mg |
| Sodium carboxymethyl starch | 4.5 mg |
| Magnesium stearate | 0.5 mg |
| Talc | 1.0 mg |
| Total | 120.0 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, starch, and magnesium stearate are blended, passed through a No. 20 mesh U.S. sieve, and filled into hard gelatin capsules in 150 mg quantities.

Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Example 7

Suspensions, each containing 50 mg of medicament per 5.0 mL dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |

(continued)

| Ingredient | Amount |
|---|---|
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Example 8

A formulation may be prepared as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, starch, and magnesium stearate are blended, passed through a No. 20 mesh U.S. sieve, and filled into hard gelatin capsules in 425.0 mg quantities.

Example 9

A formulation may be prepared as follows:

| Ingredient | Quantity |
|---|---|
| Active Ingredient | 5.0 mg |
| Corn Oil | 1.0 mL |

Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. *See, e.g.,* U.S. Patent 5,023,252, issued June 11, 1991, herein incorporated by reference in its entirety. Such patches may be constructed for continuous, pulsatile, or on demand delivery of pharmaceutical agents.

Other suitable formulations for use in the present invention can be found in *Remington's Pharmaceutical Sciences,* edited by E. W. Martin (Mack Publishing Company, 18th ed., 1990).

Biological Examples

Example 1

β2-Adrenergic Receptor *In Vitro* Functional Assay

The β2-adrenergic receptor functional activity of compounds of the invention was tested follows. J

Cell Seeding and Growth:

Primary bronchial smooth muscle cells from a 21 yr. old male (Clonetics, San Diego, CA) were seeded at 50,000 cells/well in 24-well tissue culture plates. The media used was Clonetic's SmBM-2 supplemented with hEGF, Insulin, hFGF, and Fetal Bovine Serum. Cells were grown two days at 37°C, 5% $CO_2$ until confluent monolayers were seen.

Agonist Stimulation of Cells

The media was aspirated from each well and replaced with 250 ml fresh media containing 1mM IBMX, a phospodiesterase inhibitor (Sigma, St Louis, MO). Cells were incubated for 15 minutes at 37 °C, and then 250 ml of agonist at appropriate concentration was added. Cells were then incubated for an additional 10 minutes. Media was aspirated and 500 ml cold 70% EtOH was added to cells, and then removed to an empty 96-well deep-well plate after about 5 minutes. This step was then repeated. The deep-well plate was then spun in a speed-vac until all EtOH dried off, leaving dry pellets. cAMP (pmol/well) was quantitated using a cAMP ELISA kit from Stratagene (La Jolla, CA). $EC_{50}$ curves were generated using the 4-parameter fit equation:

$$y = (a-d)/(1+ (x/c)^b) + d,$$

where,

y = cpm a = total binding $c = IC_{50}$
x = [compound] d = NS binding b = slope
Fix NS binding and allow all other parameters to float.

Example 2

β2-Adrenergic Receptor *In Vitro* Radioligand Binding Assay

The β1/2-adrenergic receptor binding activity of compounds of the invention can be tested follows. SF9 cell membranes containing either β1 or β2-adrenergic receptor (NEN, Boston, MA) were incubated with 0.07 nM $^{125}$I-iodocyanopindolol (NEN, Boston, MA) in binding buffer containing 75mM Tris-HCl (pH 7.4), 12.5 mM $MgCl_2$ and 2 mM EDTA and varying concentrations of test compounds or buffer only (control) in 96-well plates. The plates were incubated at room temperature with shaking for 1 hour. The receptor bound radioligand was harvested by filtration over 96-well GF/B filter plates (Packard, Meriden, CT) pre-blocked with 0.3%polyethylenimine and washed twice with 200μl PBS using cell harvester. The filters were washed thrice with 200μl PBS using cell harvester and then resuspended in 40μl scintillation cocktail. The filter-bound radioactivity was measured with a scintillation counter and $IC_{50}$ curves are generated using the standard 4-parameter fit equation described above.

## Claims

**1.** A compound of the formula:

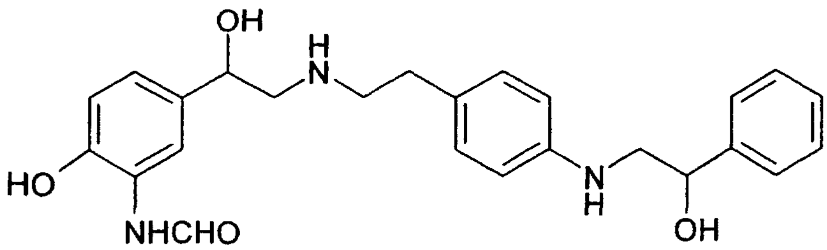

or a pharmaceutically acceptable salt thereof.

**2.** A compound of the formula:

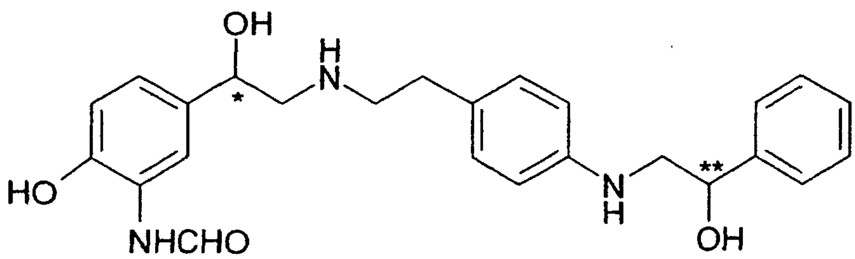

wherein the stereochemistry at *C and **C is (*RS*) and (*RS*), (*R*) and (*R*), (*R*) and (*S*), (*S*) and (*R*), or(*S*) and (*S*); or a pharmaceutically acceptable salt thereof.

**3.** The compound of Claim 2, wherein the stereochemistry at *C is (*R*) and the stereochemistry at **C is (*R*).

**4.** The compound of Claim 2, wherein the stereochemistry at *C is (*R*) and the stereochemistry at **C is (*S*).

**5.** A pharmaceutical composition comprising a pharmaceutically acceptable carrier and a therapeutically effective amount of a compound of any one of Claims 1 to 4.

**6.** The pharmaceutical composition of Claim 5, wherein the pharmaceutical composition further comprises a therapeutically effective amount of a steroidal anti-inflammatory drug.

**7.** A compound as claimed in any one of Claims 1 to 4, for use as a medicament.

**8.** A compound as claimed in Claim 7, for use as a medicament in the treatment of a respiratory disease, nervous system injury, premature labour, or a metabolic disorder.

**9.** A compound as claimed in Claim 8, in which the respiratory disease is asthma, chronic obstructive pulmonary disease or bronchitis, and the metabolic disorder is obesity or diabetes.

**10.** A pharmaceutical composition as claimed in Claims 5 or 6, for use as a medicament.

**11.** A pharmaceutical composition as claimed in Claim 10, for use as a medicament in the treatment of a respiratory disease, nervous system injury, premature labour, or a metabolic disorder.

**12.** A pharmaceutical composition as claimed in Claim 11, in which the respiratory disease is asthma, chronic obstructive pulmonary disease or bronchitis, and the metabolic disorder is obesity or diabetes.

**13.** Use of a compound as claimed in any one of Claims 1 to 4 in the manufacture of a medicament for treating a respiratory disease, nervous system injury, premature labour, or a metabolic disorder.

**14.** Use as claimed in Claim 13, in which the respiratory disease is asthma, chronic obstructive pulmonary disease or bronchitis, and the metabolic disorder is obesity or diabetes.

**Patentansprüche**

**1.** Eine Verbindung der Formel:

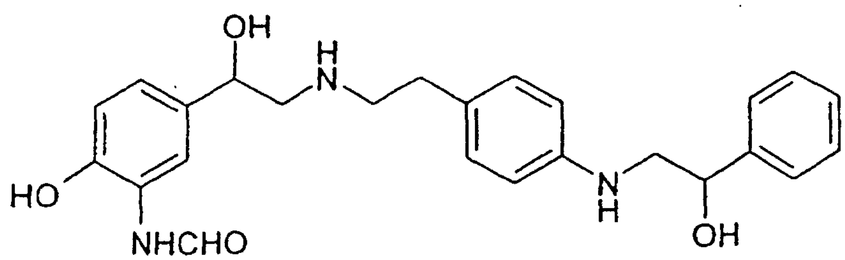

oder ein pharmazeutisch annehmbares Salz davon.

**2.** Eine Verbindung der Formel:

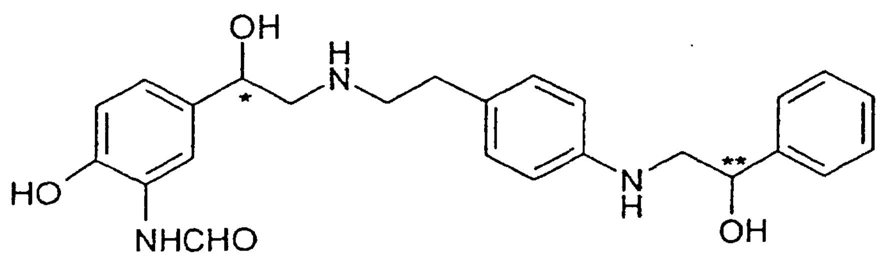

,

wobei die Stereochemie am *C und **C (RS) und (RS), (R) und (R), (R) und (S), (S) und (R), oder (S) und (S) ist, oder ein pharmazeutisch annehmbares Salz davon.

**3.** Die Verbindung nach Anspruch 2, wobei die Stereochemie am *C (R) ist und die Stereochemie am **C (R) ist.

**4.** Die Verbindung nach Anspruch 2, wobei die Stereochemie am *C (R) ist und die Stereochemie am **C (S) ist.

**5.** Eine pharmazeutische Zusammensetzung, die einen pharmazeutisch annehmbaren Träger und eine therapeutisch wirksame Menge einer Verbindung nach irgendeinem der Ansprüche 1 bis 4 enthält.

**6.** Die pharmazeutische Zusammensetzung nach Anspruch 5, wobei die pharmazeutische Zusammensetzung ferner eine therapeutisch wirksame Menge eines steroidalen Antiphlogistikums enthält.

**7.** Eine wie in irgendeinem der Ansprüche 1 bis 4 beanspruchte Verbindung zur Verwendung als ein Medikament.

**8.** Eine wie in Anspruch 7 beanspruchte Verbindung zur Verwendung als ein Medikament zur Behandlung einer Atemwegserkrankung, einer Nervensystemverletzung, von vorzeitiger Wehentätigkeit oder einer metabolischen Störung.

**9.** Eine wie in Anspruch 8 beanspruchte Verbindung, wobei die Atemwegserkrankung Asthma, chronische obstruktive Lungenerkrankung oder Bronchitis ist und die metabolische Störung Fettleibigkeit oder Diabetes ist.

**10.** Eine wie in den Ansprüchen 5 oder 6 beanspruchte pharmazeutische Zusammensetzung zur Verwendung als ein Medikament.

11. Eine wie in Anspruch 10 beanspruchte pharmazeutische Zusammensetzung zur Verwendung als ein Medikament bei der Behandlung einer Atemwegserkrankung, einer Nervensystemverletzung, von vorzeitiger Wehentätigkeit oder einer metabolischen Störung.

12. Eine wie in Anspruch 11 beanspruchte pharmazeutische Zusammensetzung, wobei die Atemwegserkrankung Asthma, chronische obstruktive Lungenerkrankung oder Bronchitis ist und die metabolische Störung Fettleibigkeit oder Diabetes ist.

13. Die Verwendung einer wie in irgendeinem der Ansprüche 1 bis 4 beanspruchten Verbindung bei der Herstellung eines Medikaments zur Behandlung einer Atemwegserkrankung, einer Nervensystemverletzung, von vorzeitiger Wehentätigkeit oder einer metabolischen Störung.

14. Die wie in Anspruch 13 beanspruchte Verwendung, wobei die Atemwegserkrankung Asthma, chronische obstruktive Lungenerkrankung oder Bronchitis ist und die metabolische Störung Fettleibigkeit oder Diabetes ist.

**Revendications**

1. Composé de formule :

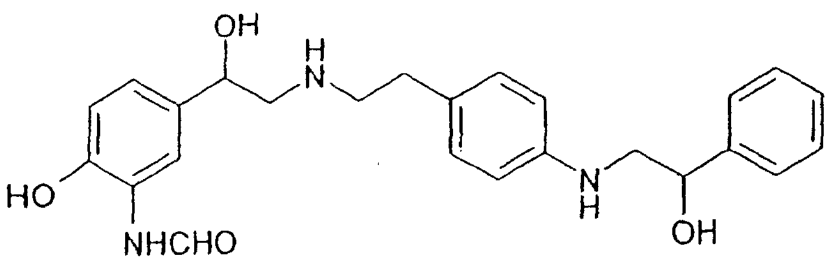

ou sel acceptable du point de vue pharmaceutique de celui-ci.

2. Composé de formule :

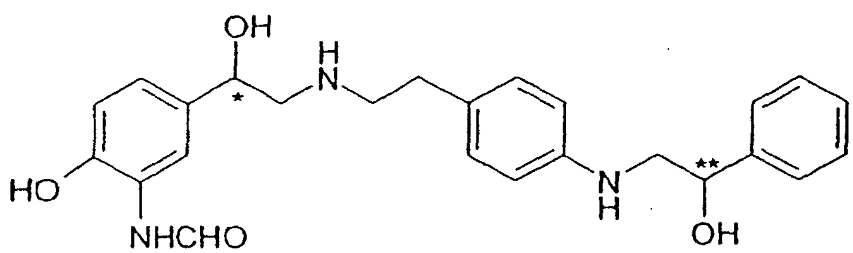

dans laquelle la stéréochimie en *C et **C est (RS) et (RS), (R) et (R), (R) et (S), (S) et (R), ou (S) et (S) ; ou sel acceptable du point de vue pharmaceutique de celui-ci.

3. Composé selon la revendication 2, dans lequel la stéréochimie en *C est (R) et la stéréochimie en **C est (R).

4. Composé selon la revendication 2, dans lequel la stéréochimie en *C est (R) et la stéréochimie en **C est (S).

5. Composition pharmaceutique comprenant un support acceptable du point de vue pharmaceutique et une quantité efficace du point de vue thérapeutique d'un composé selon l'une quelconque des revendications 1 à 4.

6. Composition pharmaceutique selon la revendication 5, dans laquelle la composition pharmaceutique comprend

de plus une quantité efficace du point de vue thérapeutique d'un médicament anti-inflammatoire stéroïdien.

**7.** Composé selon l'une quelconque des revendications 1 à 4, utile comme médicament.

**8.** Composé selon la revendication 7, utile comme médicament dans le traitement de maladie respiratoire, de lésion du système nerveux, de travail prématuré ou de trouble métabolique.

**9.** Composé selon la revendication 8, dans lequel la maladie respiratoire est l'asthme, l'insuffisance respiratoire obstructive chronique ou la bronchite, et le trouble métabolique est l'obésité ou le diabète.

**10.** Composition pharmaceutique selon la revendication 5 ou la revendication 6, utile comme médicament.

**11.** Composition pharmaceutique selon la revendication 10, utile comme médicament dans le traitement de maladie respiratoire, de lésion du système nerveux, de travail prématuré ou de trouble métabolique.

**12.** Composition pharmaceutique selon la revendication 11, dans laquelle la maladie respiratoire est l'asthme, l'insuffisance respiratoire obstructive chronique ou la bronchite, et le trouble métabolique est l'obésité ou le diabète.

**13.** Utilisation d'un composé selon l'une quelconque des revendications 1 à 4, dans la fabrication d'un médicament destiné à traiter une maladie respiratoire, une lésion du système nerveux, un travail prématuré ou un trouble métabolique.

**14.** Utilisation selon la revendication 13, dans laquelle la maladie respiratoire est l'asthme, l'insuffisance respiratoire obstructive chronique ou la bronchite, et le trouble métabolique est l'obésité ou le diabète.

*FIG.* 1

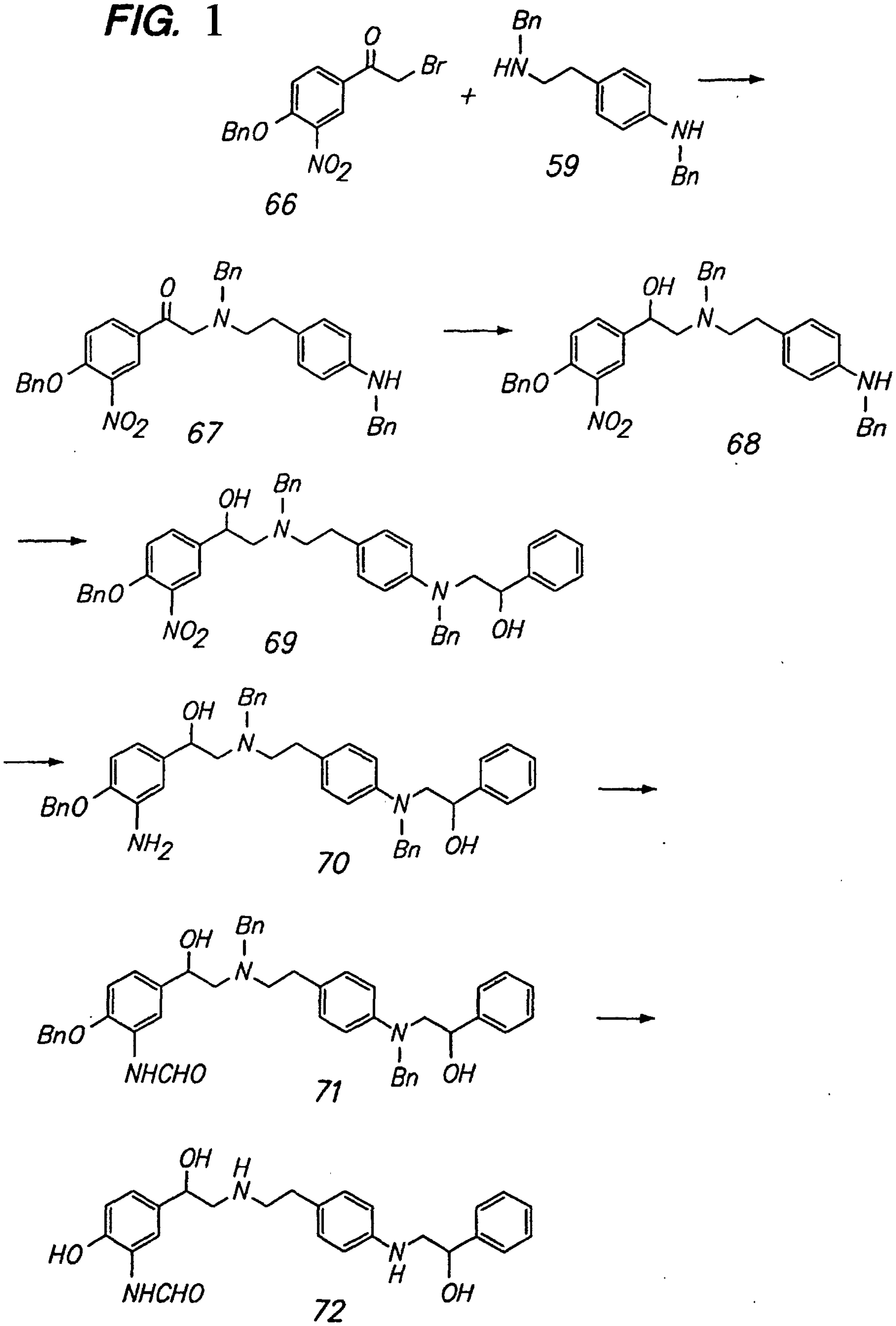

66 + 59 → 67 → 68 → 69 → 70 → 71 → 72